# FASCICULE DE BREVET EUROPEEN

(11) **EP 1 216 011 B1**
(45) Date de publication et mention de la délivrance du brevet: **24.05.2006**
(21) Numéro de dépôt: 00964373.5
(22) Date de dépôt: 26.09.2000
(51) Int. Cl.: A61F 2/34

(54) **IMPLANT ACETABULAIRE POUR PROTHESE DE HANCHE**
PFANNENIMPLANTAT FÜR EINE HÜFTPROTHESE
ACETABULAR IMPLANT FOR HIP PROSTHESIS

(30) Priorité: 28.09.1999 FR 9912085
(43) Date de publication de la demande: 26.06.2002
(73) Titulaire: Depuy (Ireland) Limited, Ringaskiddy, County Cork (IE); Descamps, Loys, 06000 Nice (FR); Derhi, Guy, 06330 Roquefort les Pins (FR); Maestro, Michel, 06200 Nice (FR); Puch, Jean-Marc, 06100 Nice (FR); Remi, Marcel, 06200 Nice (FR)
(72) Inventeur: DESCAMPS, Loys, F-06000 Nice (US); DERHI, Guy, F-06330 Roquefort les Pins (FR); MAESTRO, Michel, F-06200 Nice (FR); PUCH, Jean-Marc, F-06100 NICE (FR); REMI, Marcel, F-06200 Nice (FR); MASELLI, Alain, F-73000 Barberaz (FR); ZANELLO, Sylvain, F-69800 Saint Priest (FR)
(74) Mandataire: Jacobson, Claude
(86) Numéro de dépôt international: PCT/FR2000/002657
(87) Numéro de publication internationale: WO 2001/024740

(56) Documents cités:
- DE-A- 19 731 442
- FR-A- 2 660 546
- FR-A- 2 686 502
- FR-A- 2 710 836
- FR-A- 2 770 769

## Description

La présente invention a pour objet un implant acétabulaire pour prothèse de hanche, du type comprenant une cupule adaptée pour recevoir un insert librement articulé dans la cupule, équipée d'expansions iliaques et d'une obturatrice pour fixation osseuse.

Cet implant est de première intention et « de révision » c'est à dire qu'il peut être mis en place non seulement pour la première fois, mais aussi si la cavité osseuse a besoin d'être retouchée ou améliorée, en d'autres termes si la pathologie fait apparaître des défauts osseux nécessitant des greffons de moyenne importance. En d'autres termes, cet implant peut être utilisé pour une révision tant que la cavité cotyloïdienne n'est pas détruite à plus de 50%.

Le brevet français 93 .12.097 (2.710.836) décrit un implant acétabulaire de ce type pour prothèse totale de hanche, dans lequel la cupule a une forme sphérique, géométrie qui est généralement utilisée pour de tels implants. Toutefois une telle configuration n'est pas particulièrement bien adaptée à l'anatomie de la cavité acétabulaire d'une hanche, dont la paroi supérieure s'avance d'avantage que la paroi inférieure. Il en résulte une tenue mécanique qui peut laisser à désirer.

En outre dans les implants connus, les pattes iliaques comportent généralement une partie rectiligne directement raccordée au bord d'ouverture de la cupule. Il en résulte un manque d'adaptation à l'anatomie à cet endroit, en particulier au sourcil cotyloïdien, susceptible d'affecter le bon ancrage de la prothèse.

De nombreux implants connus comportent une cupule à surface lisse en contact avec la paroi du cotyle, parfois équipée de pointes d'ancrage, de sorte que leur tenue mécanique peut s'en trouver à la longue compromise. Pour remédier à ces insuffisances on a proposé par exemple de perforer la paroi du cotyle (brevet USA 3.740.769, brevet allemand 32.05.526). Toutefois l'expérience a montré que ces aménagements ne donnent pas entière satisfaction.

L'invention a donc pour but de réaliser un implant acétabulaire agencé de manière à lui assurer une excellente fixation per et post opératoire, aussi bien à moyen qu'à long terme.

On a déjà proposé dans FR-A-2 770 769, de prolonger la partie équatoriale d'une cupule hémisphérique recevant un insert, par un prolongement cylindrique dont une partie est tronquée et qui s'étend sur la quasi-totalité de la périphérie équatoriale de la cupule sphérique, dans le but, grâce à la présence de stries et de fentes situées dans cette zone prolongée, d'implanter et de fixer la cupule sans l'aide de vis. Une telle réalisation peut cependant se trouver exposée à des efforts importants en cas de mouvements extrêmes du composant fémoral de la prothèse.

L'invention se propose également de résoudre ces inconvénients et a pour objet un implant acétabulaire pour prothèse de hanche, comprenant une cupule adaptée pour recevoir un insert librement articulé dans la cupule, ladite cupule étant constituée d'une partie hémisphérique prolongée par une partie cylindrique, caractérisé en ce que la partie cylindrique s'étend sur sensiblement une demi-circonférence d'un bord équatorial de la partie hémisphérique, et est délimitée par un plan incliné sur le plan équatorial de la partie hémisphérique, ce plan incliné se prolongeant au-delà de la partie cylindrique par un bord tronqué de la partie hémisphérique situé dans le même plan incliné, ladite partie cylindrique étant équipée d'expansions iliaques, et ladite partie hémisphérique étant équipée, sur ledit bord tronqué, d'une expansion obturatrice d'ancrage osseux.

Ainsi la partie cylindrique, dont la largeur et l'étendue angulaire sont convenablement déterminées, prolonge la cupule dans la zone des expansions iliaques, c'est à dire dans la partie supérieure de la cavité cotyloïdienne. Cette partie cylindrique est donc en contact avec la paroi osseuse jusqu'au bord de la cavité acétabulaire, avec laquelle elle assure corrélativement un meilleur contact qu'une cupule simplement hémisphérique. En d'autres termes, la partie supérieure de l'implant recouvre étroitement la paroi osseuse ce qui diminue considérablement le risque de luxation dans les positions angulaires limites de la tige fémorale associée et de l'insert articulé dans la cupule.

L'avantage de la troncature de la partie sphérique réalisée dans la zone inférieure de l'implant réside dans le fait qu'elle empêche, dans la position extrême de l'insert en flexion-rotation interne ou en extension-rotation externe, la sortie de celui-ci à l'extérieur de la cupule par effet de came. Le col de la tige fémorale ne peut plus en effet venir en butée sur le bord inférieur d'ouverture de la cupule et risquer alors d'entraîner l'éjection de l'insert.

Dans la position extrême en flexion, le col de la tige fémorale ne risque pas de venir en butée contre la partie cylindrique de la cupule, dont la largeur est convenablement choisie à cet effet.

En définitive, cette géométrie de l'implant acétabulaire réduit très considérablement le risque de luxation dans les deux positions extrêmes opposées de l'insert et de la tige fémorale associée, tout en favorisant un excellent débattement angulaire. De plus, la géométrie de la cupule lui permet de respecter au maximum l'anatomie de la cavité acétabulaire.

Suivant une autre caractéristique de l'invention, les expansions iliaques comportent une extrémité rectiligne rapportée au bord de la cupule, prolongée par une partie courbée dont la courbure est adaptée au sourcil cotyloïdien d'une cavité acétabulaire, une partie rectiligne puis une partie incurvée prolongeant ladite partie courbée ; enfin une partie rectiligne finale raccordée à la partie incurvée et dans laquelle est agencé un trou de passage d'une vis d'ancrage osseux.

Avantageusement les deux parties rectilignes extrêmes délimitent entre elles un angle d'environ 45 degrés de préférence.

Cette géométrie permet aux expansions iliaques de s'adapter étroitement à l'anatomie osseuse à cet endroit, près du bord de la cavité acétabulaire, au besoin lui-même modelé.

Suivant une autre particularité de l'invention, une macrostructure à reliefs favorisant un ancrage primaire et une ostéointégration est usinée sur la surface externe de la cupule comprise sensiblement entre ledit plan incliné délimitant l'ouverture de la cupule et une calotte sphérique délimitant le fond de la cupule, et cette macrostructure ainsi que la calotte sont revêtus d'hydroxyapatite de calcium.

Cette macrostructure, dont la géométrie est convenablement choisie pour n'être ni trop lisse ni trop agressive, permet un meilleur ancrage primaire et à long terme de la cupule, par repousse osseuse en assurant à l'implant sa stabilité à long terme.

D'autres particularités et avantages de l'invention apparaîtront au cours de la description qui va suivre, faite en référence aux dessins annexés qui en illustrent une forme de réalisation à titre d'exemple non limitatif.
La figure 1 est une vue en élévation latérale sensiblement à l'échelle, d'une forme de réalisation de l'implant acétabulaire selon l'invention, vu dans un plan sagittal.
La figure 2 est une vue en élévation dans un plan frontal de l'implant de la figure 1.
La figure 3 est une vue en élévation à échelle agrandie par rapport aux figures 1 et 2, d'une forme de réalisation industrielle de l'implant conforme à l'invention.
La figure 4 est une vue en élévation suivant la flèche K de la figure 3.
La figure 5 est une vue en élévation de l'implant acétabulaire prise suivant la direction de la flèche K de la figure 3.
La figure 6 est une vue en coupe suivant 6-6 de la figure 3.
La figure 7 est une vue en perspective à échelle agrandie d'un détail de la surface externe à macrostructure de l'implant des figures 3 à 6.
La figure 8 est une vue en coupe partielle à échelle agrandie de rainures équatoriales visibles aux figures 3 à 6.
La figure 9 est une vue en élévation à échelle agrandie d'un mode de réalisation d'une expansion iliaque rapportée à un implant selon l'une des figures 1 à 7.
La figure 10 est une vue en élévation latérale de l'expansion iliaque de la figure 9.
La figure 11 est une vue en perspective de l'expansion iliaque des figures 9 et 10.
La figure 12 est une vue en élévation dans un plan frontal de l'implant selon l'invention ainsi que de l'extrémité supérieure de la tige fémorale associée, représentée en abduction-extension.
La figure 13 est une vue en élévation analogue à la figure 12, représentant la position extrême pouvant être prise par la tige fémorale par rapport à l'implant acétabulaire en adduction-flexion.

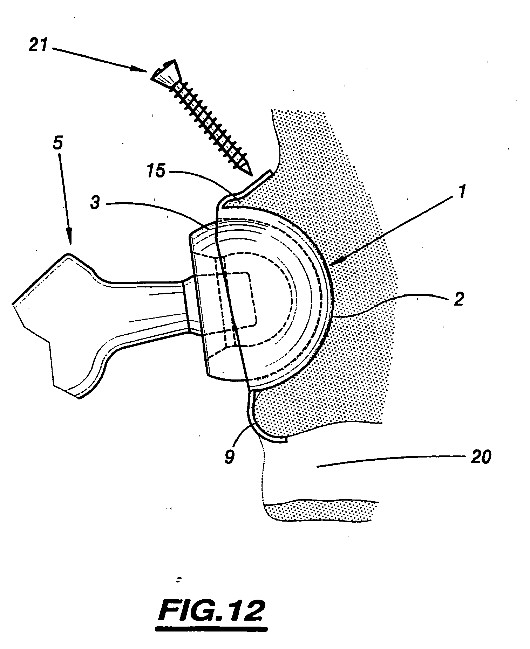

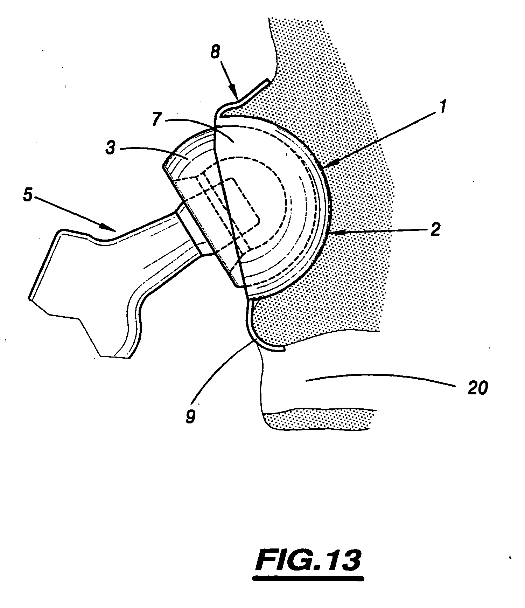

L'implant acétabulaire 1 illustré aux dessins est destiné à une prothèse totale de hanche, dont seule la partie proximale 5 de la tige fémorale a été représentée aux figures 12 et 13.

L'implant 1 comprend une cupule 2 adaptée pour recevoir un insert 3 librement articulé dans la cupule et dans lequel peut être introduite une tête (figures 12 et 13) rapportée à l'extrémité du col de la partie proximale 5.

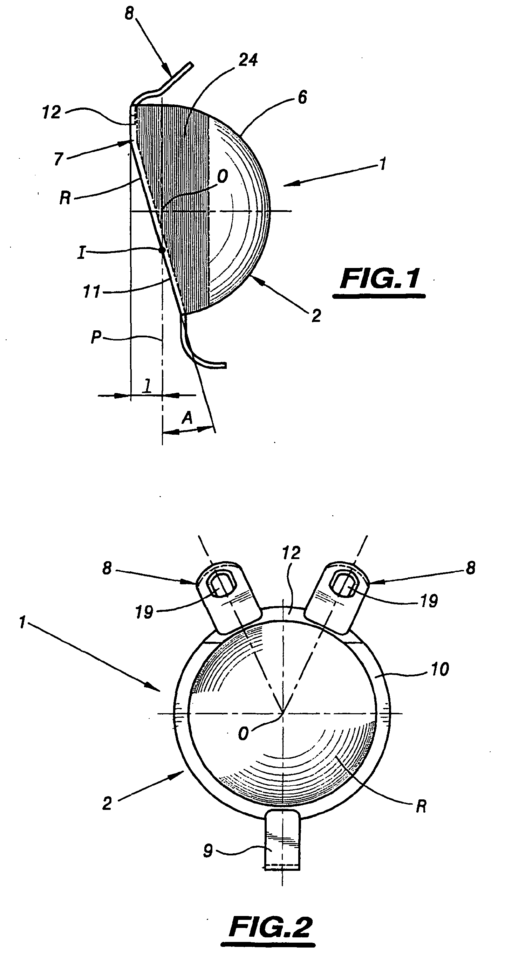

La cupule 2 est constituée d'une partie hémisphérique 6 délimitée par un plan équatorial P (figure 1) prolongée par une partie cylindrique 7 à laquelle sont fixées deux « expansions » iliaques 8. En regard de celles-ci une obturatrice 9, formée par un crochet est rapportée au bord inférieur d'ouverture de la partie hémisphérique 6 de la cupule 2, en une position diamétralement opposée à un emplacement situé au milieu d'un intervalle entre les expansions 8.

La partie cylindrique 7 s'étend au delà du plan équatorial P, sur une partie de la circonférence du bord d'ouverture de la cupule 6, à savoir sensiblement sur une demi circonférence. De manière plus précise, la partie cylindrique 7 s'étend en fait sur un secteur angulaire sensiblement supérieur à une demi circonférence. On voit à la figure 1 que cette partie 7 est délimitée par un plan R incliné sur le plan équatorial P, et qui se prolonge au delà de la partie cylindrique 7 par un bord tronqué 11 de la partie hémisphérique 6, ce bord tronqué 11 se situant dans le même plan incliné R. Ainsi est réalisée une sorte de troncature de la partie hémisphérique 6 s'étendant depuis l'intersection I entre le plan incliné R et le plan équatorial P, jusqu'au bord inférieur de la cupule 2 et à l'obturatrice 9.

La partie cylindrique 7 possède, dans la direction radiale, une largeur I appropriée à l'anatomie du cotyle, et le plan R délimite avec le plan équatorial P un angle A d'environ 15 degrés, dont le sommet est l'intersection I. Le plan incliné R intersecte le plan équatorial P entre le centre O de la partie hémisphérique 6 et son bord attenant à l'obturatrice 9.

Le bord extérieur de la partie cylindrique 7 est tronqué de manière à délimiter un chanfrein 12 s'étendant parallèlement au plan équatorial P et sur lequel viennent se fixer les expansions iliaques 8.

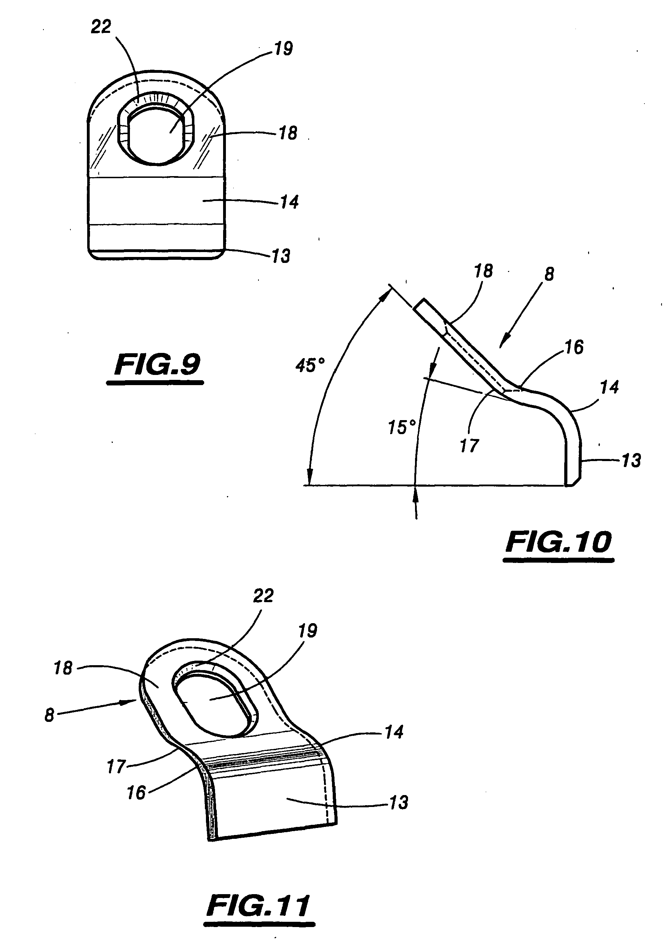

Chaque expansion iliaque 8 est adaptable à la surface ossseuse individuelle. Elle comporte une extrémité rectiligne 13 rapportée au bord de la cupule 2, prolongée par une partie courbée 14 dont la courbure est adaptée au sourcil cotyloïdien 15 d'une cavité acétabulaire (figure 12), une partie rectiligne 16 puis une seconde partie incurvée 17 (figures 9 à 11) prolongeant ladite partie courbée 16 ; enfin chaque expansion 8 se termine par une partie rectiligne finale 18 dans laquelle est agencé un trou 19, avantageusement de forme oblongue, pour le passage d'une vis éventuelle 21 d'ancrage osseux (figure 12).

La partie rectiligne finale 18 est avantageusement inclinée d'environ 45 degrés sur la partie rectiligne initiale 13, tandis que la partie rectiligne intermédiaire 16 peut être inclinée de 15 degrés environ sur la perpendiculaire à la partie 13. Le trou oblong 19 est avantageusement muni d'un lamage 22 sur son pourtour, permettant une polyorientation de la vis d'ancrage 21.

La courbure de la partie incurvée 14, adaptée au passage du sourcil cotyloïdien, et l'angulation de la partie rectiligne 18 à 45 degrés par rapport à la patte 13 garantissent la proximité entre les expansions 8 et la surface osseuse. Ainsi, ces deux particularités assurent un excellent respect de l'anatomie locale par les expansions iliaques 8.

Une macrostructure 24 à reliefs 28 est usinée sur une partie de la surface externe de la cupule 2. Cette partie recouverte par la macrostructure 24 est délimitée sensiblement par le bord 10 d'ouverture de la cupule 2 et une calotte sphérique 25 formant le fond de la cupule 2. Cette macrostructure 24 ainsi que la calotte 25 sont revêtus d'une couche d'hydroxyapatite de calcium contribuant, avec la macrostructure 24, à la repousse osseuse et à l'ostéointégration.

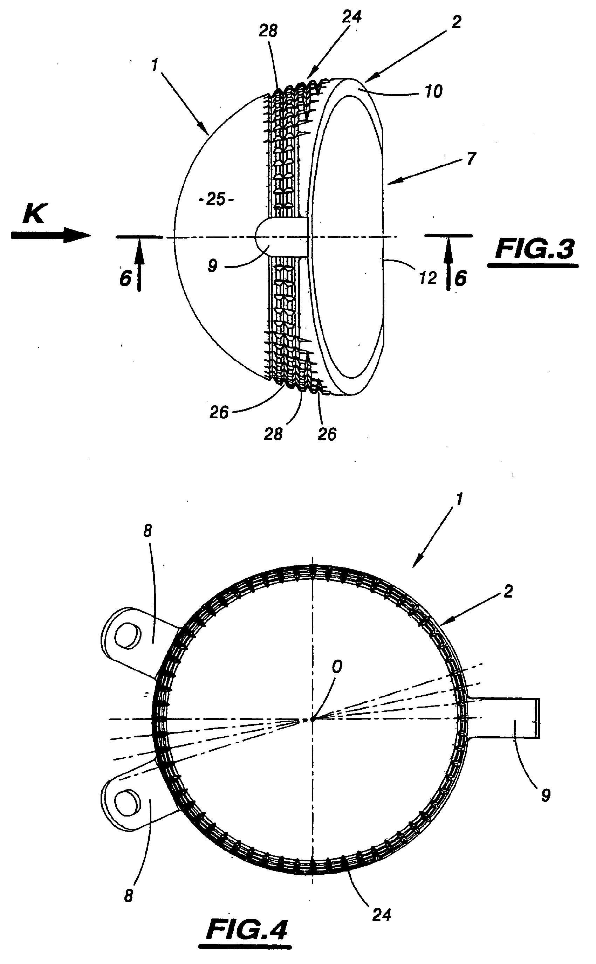

La macrostructure 24 est séparée du bord libre 10 de la cupule 2 par une bande lisse 26 de faible largeur. La macrostructure 24 délimite un ensemble de rainures équatoriales 26 situées dans des plans équatoriaux parallèles au plan P, et un ensemble de rainures méridiennes 27 perpendiculaires aux rainures équatoriales 26. L'ensemble de ces rainures 26 et 27 forme donc une sorte de quadrillage, délimitant, aux intersections entre les rainures 26 et 27, dont les sections transversales ont un profil en V (figures 3 à 8), une série de reliefs 28 ayant chacun une base rectangulaire.

Les flancs 26a des rainures équatoriales 26 peuvent être avantageusement mais non limitativement inclinés d'un angle B de 30 degrés environ sur un plan médian M passant par le fond de chaque rainure 26, dont l'ouverture angulaire est ainsi de 60 degrés et dont la profondeur peut être par exemple de 1 mm environ.

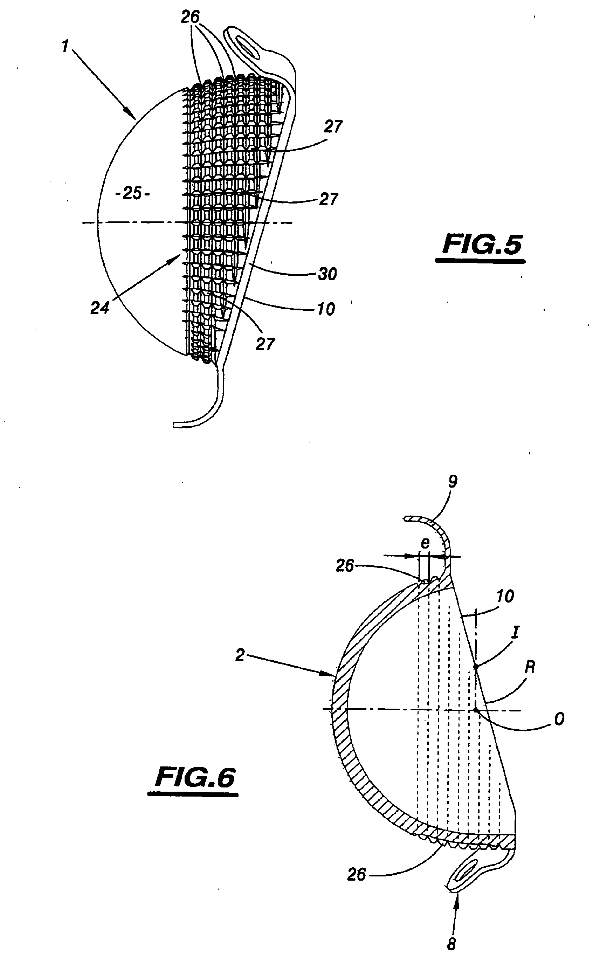

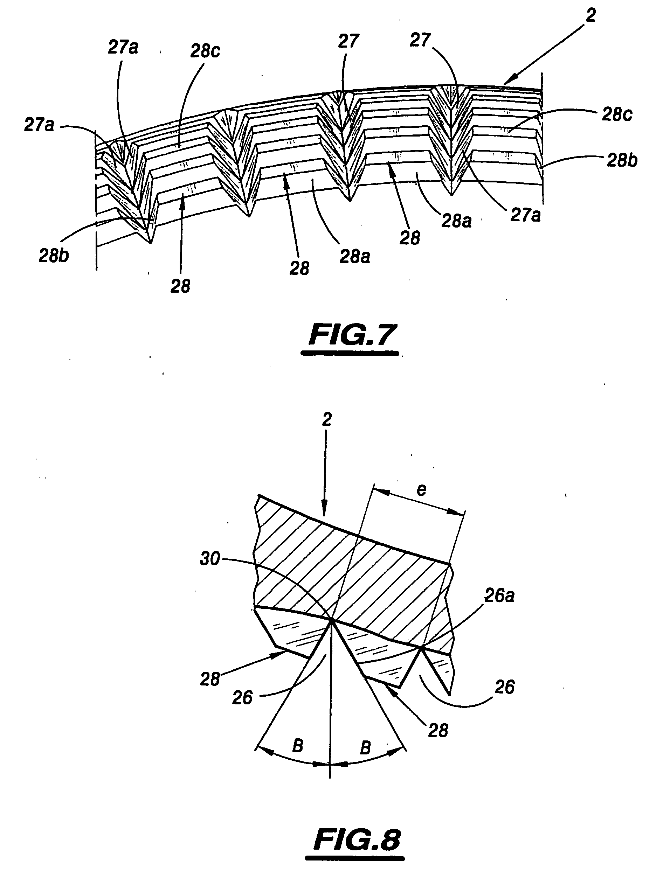

Il en est de même pour les flancs 27a des rainures méridiennes 27 (figure 7). Par ailleurs, également à titre d'exemple numérique non limitatif, les arêtes formant le fond des rainures équatoriales 26 peuvent être espacées d'un intervalle e de 2mm environ (figures 6 et 8), le nombre de ces rainures équatoriales 26 variant avec la taille de la cupule 2 .

Enfin les rainures méridiennes 27 sont espacées deux à deux angulairement avantageusement de 6 degrés environ (figure 7), de sorte que leur nombre total sur le pourtour de la cupule 2 est de 60. Ce nombre total est constant quelle que soit la taille de la cupule.

On voit aux figures 3 et 7 que chacun des reliefs 28 présente des profils transversal et longitudinal, dont les flancs transversaux 28a délimitent des rainures équatoriales 26, tandis que leurs flancs longitudinaux 28b délimitent des rainures méridiennes 27. Tous ces flancs sont raccordés à un plat 28c.

La cupule 2, non cimentée, peut être réalisée par exemple en acier inoxydable selon la norme ISO 53 32-1, recouverte d'hydroxyapatite de calcium (HAC).

La mise en place de l'implant acétabulaire 1 qui vient d'être décrit s'effectue comme représenté à la figure 12: ses dimensions sont choisies de manière que le diamètre de la cupule 2 soit légèrement supérieur au diamètre du cotyle naturel dans lequel elle doit être implantée par effet de « press fit » c'est à dire par impaction. En même temps, les expansions iliaques 8 se mettent en place en chevauchant le sourcil cotyloïdien 15 tandis que l'obturatrice 9 vient s'introduire dans le trou obturateur 20. Si nécessaire les vis 21 sont implantées à travers les trous 19 suivant l'orientation appropriée afin de trouver le meilleur ancrage osseux, ces vis 21 pouvant être du type soit à os spongieux soit à corticale.

La stabilité immédiate, per et post opératoire de la cupule 2, est assurée par la combinaison des éléments suivants :
- l'impaction en « press fit » de l'implant 1 dans le cotyle naturel,
- la présence de la macrostructure 24 revêtue d'hydroxyapatite,
- le vissage (éventuel) des expansions iliaques par les vis 21,
- la mise en position et le maintien de la cupule 2 par l'obturatrice 9.

La stabilité de l'implant à moyen et long terme est obtenue par l'ostéoconduction induite par le revêtement d'hydroxyapatite de calcium, et par l'ostéointégration au coeur de la macrostructure 24 ainsi qu'éventuellement par l'action des expansions iliaques 8 et de l'obturatrice 9.

Ainsi tous ces éléments garantissent une excellente fixation primaire et secondaire de l'implant 1, et par conséquent une durée de vie optimale.

Les expansions iliaques 8 adaptables à l'os, dont elles épousent la surface osseuse, et l'obturatrice 9 permettent de pincer la branche ischio-pubienne.

La présence de la bande lisse 30 évite de denteler le plan équatorial P par le bord de la macrostructure 24. Par ailleurs, les valeurs numériques mentionnées ci-dessus, quoique non limitatives, se sont révélées aux essais comme fournissant les résultats les plus avantageux en termes d'amélioration de l'efficacité de la fixation primaire et secondaire à long terme de l'implant. Ainsi le fait de limiter le nombre total de rainures méridiennes 28 à 60 quelle que soit la taille de l'implant acétabulaire, permet de réaliser pour les grandes tailles, de grands damiers avec des reliefs importants 28 et qui favorisent l'accrochage à la paroi osseuse. La macrostructure 24 est avantageusement usinée sur la cupule 2 et non rapportée, ce qui s'est révélé plus efficace en termes d'ostéointégration.

Par ailleurs il est avéré inutile d'agencer une macrostructure 24 sur la calotte sphérique 25 qui vient en contact avec le fond du cotyle naturel. En effet les contraintes subies à ce niveau sont faibles, alors qu'elles sont maximales dans la zone supérieure du cotyle, les efforts exercés diminuant dans les zones postérieure et antérieure de la cavité.

En résumé, la géométrie de la cupule 2, hémisphérique surmontée d'une partie cylindrique 7, avec troncature à 15 degrés par le plan R, permet un excellent débattement angulaire jusque dans les positions limites en évitant des risques de luxation dans ces positions extrêmes, tout en respectant en outre au maximum l'anatomie de la cavité acétabulaire.

La géométrie des expansions iliaques 8 leur assure un contact optimal avec la surface osseuse, grâce en particulier au pliage de ces expansions au niveau de leur partie incurvée 14.

L'association de la macrostructure 24 dont la géométrie est bien définie comme exposée ci-dessus, et d'un revêtement d'hydroxyapatite de calcium, assure, d'une part une bonne pénétration des trabécules osseux au sein de la macrostructure 24, c'est à dire favorise une bonne ostéointégration, et d'autre part garantit la stabilité de l'implant acétibulaire dans le temps.

L'invention n'est pas limitée aux modes de réalisation décrits et peut comporter de nombreuses variantes d'exécution. Ainsi les dimensions et la géométrie des reliefs 28 définis par la macrostructure 24 pourraient différer sensiblement de l'exemple décrit, de même que l'inclinaison par exemple du plan R sur le plan équatorial P.

## Revendications

1. Implant acétabulaire (1) pour prothèse de hanche, comprenant une cupule (2) adaptée pour recevoir un insert (3) librement articulé dans la cupule, ladite cupule étant constituée d'une partie hémisphérique (6) prolongée par une partie cylindrique (7), **caractérisé en ce que** la partie cylindrique (7) s'étend sur sensiblement une demi-circonférence d'un bord équatorial de la partie hémisphérique (6), et est délimitée par un plan (R) incliné sur le plan équatorial (P) de la partie hémisphérique (6), ce plan incliné se prolongeant au-delà de la partie cylindrique (7) par un bord tronqué (11) de la partie hémisphérique (6) situé dans le même plan incliné (R), ladite partie cylindrique (7) étant équipée d'expansions iliaques (8), et ladite partie hémisphérique (6) étant équipée, sur ledit bord tronqué, d'une expansion obturatrice d'ancrage osseux.

2. Implant selon la revendication 1, **caractérisé en ce que** ledit plan incliné (R) délimite avec le plan équatorial (P) de la cupule (2) un angle (A) d'environ 15 degrés, qui intersecte le plan équatorial entre le centre (O) de la partie hémisphérique (6) et son bord attenant à l'obturatrice (9), la partie cylindrique (7) ayant une largeur (I) appropriée à cet effet.

3. Implant selon l'une des revendications 1 et 2, **caractérisé en ce que** les expansions iliaques (8) comportent une extrémité rectiligne (13) rapportée au bord de la cupule (2), prolongée par une partie courbée (14) dont la courbure est adaptée au sourcil cotyloïdien (15) d'une cavité acétabulaire, une partie rectiligne (16) puis une partie incurvée (17) prolongeant ladite partie courbée, et enfin une partie rectiligne finale (18) raccordée à la partie incurvée et dans laquelle est agencé un trou (19) de passage d'une vis (21) d'ancrage osseux.

4. Implant selon la revendication 3, **caractérisé en ce que** la partie rectiligne finale (18) est inclinée d'environ 45 degrés sur ladite extrémité rectiligne (13), le trou (19) est oblong et comporte un lamage (22) permettant une polyorientation de la vis (21) d'ancrage osseux.

5. Implant selon l'une des revendications 1 à 4, **caractérisé en ce qu'**une macrostructure (24) à reliefs (28) favorisant une ostéointégration est usinée sur la surface externe de la cupule (2) comprise sensiblement entre ledit plan incliné (R) délimitant l'ouverture de la cupule (2) et une calotte sphérique (25) délimitant le fond de la cupule, et cette macrostructure ainsi que la calotte sont revêtus d'hydroxyapatite de calcium.

6. Implant selon la revendication 5, **caractérisé en ce qu'**une bande lisse (30) est réservée entre le bord d'ouverture (10) de la cupule (2) et le début de la macrostructure (24) à reliefs (28).

7. Implant selon les revendications 5 et 6, **caractérisé en ce que** la macrostructure (24) délimite un ensemble de rainures équatoriales (26), situées dans des plans équatoriaux parallèles et un ensemble de rainures méridiennes (27) perpendiculaires aux rainures équatoriales, et ces différentes rainures équatoriales et méridiennes ont une section transversale en V dont les flancs (26a) sont inclinés de 30 degrés environ sur un plan médian (M) passant par le fond de la rainure (26, 27) tandis que la profondeur de ces rainures équatoriales et méridiennes est de 1 mm environ.

8. Implant selon la revendication 7, **caractérisé en ce que** les arêtes (30) formant le fond des rainures équatoriales (26) sont espacées de 2 mm environ et le nombre de ces rainures varie avec la taille de la cupule (2).

9. Implant selon la revendication 7 ou 8, **caractérisé en ce que** les rainures méridiennes (127) sont espacés deux à deux angulairement de 6 degrés environ de sorte que leur nombre total sur le pourtour de la cupule (2) est de 60, ce nombre total étant constant quelle que soit la taille de la cupule.

## Claims

1. Acetabular implant (1) for a hip prosthesis, comprising a cup (2) arranged to receive an insert (3) freely articulated in the cup, said cup being composed of a hemispherical portion (6) extended by a cylindrical portion (7), **characterised in that** the cylindrical portion (7) extends over substantially half a circumference of an equatorial edge of the hemispherical portion (6) and is delimited by a plane (R) inclined with respect to the equatorial plane (P) of the hemispherical portion (6), that inclined plane being extended beyond the cylindrical portion (7) by means of a truncated edge (11) of the hemispherical portion (6), which edge (11) is located in the same inclined plane (R), said cylindrical portion (7) being provided with iliac extensions (8), and said hemispherical portion (6) being provided, on said truncated edge, with an obturator extension for bone anchoring.

2. Implant according to claim 1, **characterised in that** said inclined plane (R) delimits, together with the equatorial plane (P) of the cup (2), an angle (A) of about 15 degrees, which intersects the equatorial plane between the centre (O) of the hemispherical portion (6) and its edge adjoining the obturator element (9), the cylindrical portion (7) having a width (I) appropriate for that purpose.

3. Implant according to one of claims 1 and 2, **characterised in that** the iliac extensions (8) comprise a rectilinear end (13) attached to the edge of the cup (2) and extended by a curved portion (14) whose curvature is matched to the cotyloid brow (15) of an acetabular cavity, a rectilinear portion (16) and then an incurvate portion (17) extending said curved portion, and finally a final rectilinear portion (18) which is attached to the incurvate portion and in which there is provided a through-hole (19) for a screw (21) for bone anchoring.

4. Implant according to claim 3, **characterised in that** the final rectilinear portion (18) is inclined about 45 degrees with respect to said rectilinear end (13), the hole (19) is oblong and comprises a countersink (22) allowing polyorientation of the screw (21) for bone anchoring.

5. Implant according to one of claims 1 to 4, **characterised in that** a macrostructure (24) having reliefs (28) promoting osseointegration is machined on the external surface of the cup (2) included substantially between said inclined plane (R) delimiting the opening of the cup (2) and a spherical cap (25) delimiting the bottom of the cup, and that macrostructure and also the cap are coated with calcium hydroxyapatite.

6. Implant according to claim 5, **characterised in that** a smooth band (30) is reserved between the edge of the opening (10) of the cup (2) and the start of the macrostructure (24) having reliefs (28).

7. Implant according to claims 5 and 8, **characterised in that** the macrostructure (24) delimits a set of equatorial grooves (26) located in parallel equatorial planes and a set of meridional grooves (27) perpendicular to the equatorial grooves, and those different equatorial and meridional grooves have a V-shaped transverse section the sides (26a) of which are inclined about 30 degrees with respect to a medial plane (M) passing through the bottom of the groove (26, 27), whilst the depth of those equatorial and meridional grooves is about 1 mm.

8. Implant according to claim 7, **characterised in that** the apices (30) forming the bottom of the equatorial grooves (26) are spaced about 2 mm apart, and the number of those grooves varies according to the size of the cup (2).

9. Implant according to claim 7 or 8, **characterised in that** the meridional grooves (127) are spaced apart from one to the next at angles of about 6 degrees so that their total number over the circumference of the cup (2) is 60, that total number being constant whatever the size of the cup.

## Patentansprüche

1. Pfannenimplantat (1) für eine Hüftprothese, eine Schale (2) umfassend, die dafür ausgebildet ist, einen frei in der Schale gelenkig gelagerten Einsatz (3) aufzunehmen, wobei die genannte Schale aus einem halbkugelförmigen Teil (6) besteht, der sich in einem zylindrischen Teil (7) fortsetzt, **dadurch gekennzeichnet, dass** sich der zylindrische Teil (7) im Wesentlichen über den halben Umfang eines äquatorialen Randes des halbkugelförmigen Teils (6) erstreckt und durch eine Ebene (R) begrenzt wird, die relativ zur Äquatorialebene (P) des halbkugelförmigen Teils (6) geneigt ist, wobei sich diese geneigte Ebene jenseits des zylindrischen Teils (7) in einem schräg abgestumpften Rand (11) des halbkugelförmigen Teils (6) fortsetzt, der in derselben geneigten Ebene (R) liegt, wobei der genannte zylindrische Teil (7) mit iliakalen Fortsätzen (8) versehen ist und der genannte halbkugelförmige Teil (6) auf dem genannten abgestumpften Rand mit einem Obturatorfortsatz zur Knochenverankerung versehen ist.

2. Implantat nach Patentanspruch 1, **dadurch gekennzeichnet, dass** die genannte geneigte Ebene (R) mit der Äquatorialebene (P) der Schale (2) einen Winkel (A) von ungefähr 15 Grad einschließt und die Äquatorialebene zwischen dem Zentrum (O) des halbkugelförmigen Teils (6) und seinem an den Obturator (9) anschließenden Rand schneidet, wobei der zylindrische Teil (7) eine diesem Zweck angepasste Breite (I) hat.

3. Implantat nach einem der Patentansprüche 1 und 2, **dadurch gekennzeichnet, dass** die iliakalen Fortsatze (8) ein geradliniges Ende (13) aufweisen, das an den Rand der Schale (2) angesetzt ist, das sich in einem gebogenen Teil (14) fortsetzt, dessen Krümmung dem Pfannendach (15) einer Hüftgelenkpfanne angepasst ist, wobei ein geradliniger Teil (16) und dann ein gekrümmter Teil (17) den genannten gebogenen Teil fortsetzen, und schließlich ein geradliniger Endabschnitt (18), der an den gekrümmten Teil angesetzt ist und in dem ein Loch (19) für den Durchtritt einer Schraube (21) zur Knochenverankerung ausgebildet ist.

4. implantat nach Patentanspruch 3, **dadurch gekennzeichnet, dass** der geradlinige Endabschnitt (18) um ungefähr 45 Grad relativ zum genannten geradlinigen Ende (13) geneigt ist, dass das Loch (19) länglich ist und eine Senkung (22) aufweist, die mehrere Orientierungen der Schraube (21) zur Knochenverankerung eriaubt.

5. Implantat nach einem der Patentansprüche 1 bis 4, **dadurch gekennzeichnet, dass** eine Makrostruktur (24) mit Reliefs (28), die die Knochenintegration fordert, auf der Außenfläche der Schale (2) ausgebildet ist, die sich im Wesentlichen zwischen der genannten geneigten Ebene (R), die die Öffnung der Schale (2) begrenzt, und einer Kugelkappe (25) befindet, die den Boden der Schale begrenzt, und dass diese Makrostruktur, sowie die Kugelkappe, mit Kalziumhydroxyapatit beschichtet sind.

6. Implantat nach Patentanspruch 5, **dadurch gekennzeichnet, dass** ein glatter Streifen (30) zwischen dem Öffnungsrand (10) der Schale (2) und dem Beginn der Makrostruktur (24) mit Reliefs (28) ausgespart wird.

7. Implantat nach den Patentansprüchen 5 und 6, **dadurch gekennzeichnet, dass** die Makrostruktur (24) eine Reihe Äquatorialrillen (26) umfasst, die sich in parallelen Äquatorialebenen befinden, und eine Reihe von Meridianrillen (27), die zu den Äquatorialrillen rechtwinklig verlaufen und dass diese verschiedenen Äquatorial- und Meridianrillen einen Querschnitt in Form eines V haben, dessen Flanken (26a) um ungefähr 30 Grad zu einer Mittelebene (M) geneigt sind, die durch den Boden der Rille (26, 27) verläuft, während die Tiefe dieser Äquatorial- und Meridianrillen ungefähr 1 mm beträgt.

8. Implantat nach Patentanspruch 7, **dadurch gekennzeichnet, dass** die Kanten (30), die den Boden der Äquatorialrillen (26) bilden, um ungefähr 2 mm voneinander beabstandet sind, und dass die Anzahl dieser Rillen mit der Größe der Schale (2) variiert.

9. Implantat nach Patentanspruch 7 oder 8, **dadurch gekennzeichnet, dass** die Meridianrillen (127) paarweise voneinander um einen Winkel von ungefähr 6 Grad beabstandet sind, so dass ihre Gesamtzahl auf dem Umfang der Schale (2) 60 beträgt, wobei diese Gesamtzahl unabhängig von der Größe der Schale konstant ist.
